(19) Europäisches Patentamt
European Patent Office
Office européen des brevets

(11) **EP 3 036 217 B1**

(12) **EUROPÄISCHE PATENTSCHRIFT**

(45) Veröffentlichungstag und Bekanntmachung des Hinweises auf die Patenterteilung:
**28.06.2017 Patentblatt 2017/26**

(51) Int Cl.:
***C07C 263/20*** *(2006.01)*   ***C07C 265/14*** *(2006.01)*

(21) Anmeldenummer: **14750749.5**

(86) Internationale Anmeldenummer:
**PCT/EP2014/067432**

(22) Anmeldetag: **14.08.2014**

(87) Internationale Veröffentlichungsnummer:
**WO 2015/024859 (26.02.2015 Gazette 2015/08)**

(54) **VERFAHREN ZUR GEWINNUNG VON ORGANISCHEN ISOCYANATEN AUS DESTILLATIONSRÜCKSTÄNDEN DER ISOCYANATHERSTELLUNG**

PROCESS FOR THE PRODUCTION OF ORGANIC ISOCYANATES FROM DISTILLATION RESIDUES OF THE ISOCYANATE PRODUCTION PROCESS

PROCÉDÉ DE COLLECTE D'ISOCYANATES ORGANIQUES À PARTIR DE RÉSIDUS DE DISTILLATION DE LA FABRICATION D'ISOCYANATE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.08.2013 EP 13180927**

(43) Veröffentlichungstag der Anmeldung:
**29.06.2016 Patentblatt 2016/26**

(73) Patentinhaber: **Covestro Deutschland AG**
**51373 Leverkusen (DE)**

(72) Erfinder:
• **TIDOW, Klaus**
**25591 Ottenbüttel (DE)**

• **HECKING, Andreas**
**40764 Langenfeld (DE)**
• **BUSCH, Jan**
**40477 Düsseldorf (DE)**
• **RICHTER, Frank**
**51373 Leverkusen (DE)**
• **STEFFENS, Friedhelm**
**51373 Leverkusen (DE)**

(74) Vertreter: **Levpat**
**c/o Covestro AG**
**Gebäude 4825**
**51365 Leverkusen (DE)**

(56) Entgegenhaltungen:
**EP-A1- 1 371 635      EP-A1- 1 518 874**
**DE-A1- 10 260 093**

**Beschreibung**

Die vorliegende Erfindung betrifft ein Verfahren zur Gewinnung eines organischen Isocyanats aus einem das Isocyanat enthaltenden Phosgenierungsprodukt.

Die Herstellung von Di- oder Polyisocyanaten wie Toluylendiisocyanat (TDI) durch Phosgenierung von Toluylendiamin (TDA) und die anschließende destillative Aufreinigung des rohen Isocyanats beziehungsweise des TDI sind allgemein bekannt. All den bekannten Verfahren zur destillativen Reinigung des rohen TDI ist gemeinsam, daß neben dem gewünschten gereinigten TDI aus der Destillation höher siedender Komponenten erhalten werden, welche mindestens einer sachgerechten Entsorgung zuführbar gemacht werden müssen. Der Stand der Technik zur Behandlung sogenannter Destillationsrückstände der TDI-Herstellung beschreibt verschiedene Verfahren. Generelle Ziele der Rückstandsbehandlung sind die Maximierung der TDI Ausbeute, die Minimierung des Mengenanfalls an Rückstand und eine möglichst sinnvolle kostengünstige und einfache Verwertung der für den TDI-Herstellprozeß nicht mehr nutzbaren Rückstandsmenge. Die Aufarbeitung von Rückstanden aus der Isocyanat Herstellung ist von steigendem wirtschaftlichem Interesse, da mit zunehmender Anlagengröße die Menge an Rückstand sowie die darin enthaltende Wertstoffmenge steigt.

Die thermisch induzierte unerwünschte Bildung von höheren Polymeren aus Diisocyanaten durch die Reaktion mit Spuren an Feuchte, Aminen sowie untereinander zu z.B. Harnstoffen, Uretdionen, Biureten, Isocyanuraten, Carbodiimden und Uretoniminen ist allgemein bekannt und vielfach in der Literatur beschrieben. Als wesentlich nachteilig an diesen unerwünschten Nebenreaktionen ist, das zum einen bei ihrer Bildung Wertstoff (TDI) verbraucht wird und es andererseits dabei zu einem unkontrollierbaren Polymeraufbau kommt welcher mit einem Viskositätsanstieg einhergeht. Dies trifft im besonderen Maße für Rückstandskonzentrationen oberhalb > 10% in TDI zu. Ebenso führt der Polymeraufbau in vielen Fällen zu in organischen Lösungsmitteln unlöslichen Verbindungen. Somit sind solche Rückstände nur unter größerer Anstrengung und in geringen Konzentrationen in verfahrenstechnisch einfach handhabbare Lösungen zu überführen. Für solche Rückstände kommen durch ihre schlechte Handhabbarkeit nur wenige aufwendige Verfahren zum Tragen. Was ferner die Wirtschaftlichkeit der Aufarbeitung solcher Rückstände in erheblichem Maße schmälert.

Zur Minimierung der Isocyanatausbeuteverluste kann der Destillationsrückstand in einen gerührten und beheizten Behälter überführt und mit hochsiedenden Kohlenwasserstoffen, vorzugsweise Bitumen, die unter den Destillationsbedingungen inert sind, vermischt werden, um möglichst vollständig das im Rückstand noch vorhandene freie Isocyanat abzudestillieren (EP 0 548 685 A2). Der verbleibende, von Isocyanat befreite Rückstand kann als rieselfähiger Feststoff ausgetragen und einer Verbrennung zugeführt werden. Nachteile dieses Verfahrens sind neben dem Einsatz eines prozessfremden Stoffes (Bitumen) Ausbeuteverluste durch Polymerisation des Isocyanats, da der Prozess hohe Verweilzeiten bei hoher Temperatur beinhaltet.

Ein weiteres Verfahren zur Isocyanat-Rückstandsabtrennung ist gekennzeichnet durch den Einsatz von Knetertrocknern (EP 0 626 368 A1). In diesem Verfahren werden die oben beschriebenen beheizten und gerührten Behälter durch Knetertrockner ersetzt. Durch Einsatz von zum Beispiel Bitumen wird wie im oben genannten Beispiel der verbleibende Rückstand als rieselfähiger Feststoff erhalten der als Brennstoff zum Beispiel in Zementwerken eingesetzt werden kann. Vorteil dieses Verfahrens gegenüber dem oben genannten ist eine Ausbeuteerhöhung, als Nachteil können die erforderlichen höheren Investitionskosten gesehen werden, bedingt durch die aufwendigere Technik. Außerdem führt der Einsatz mechanisch bewegter Teile zwangsläufig zu höherem Wartungsaufwand.

EP 0 699 659 A2 beschreibt ein Verfahren und eine Vorrichtung zur Abtrennung eines festen Rückstandes aus einer Lösung des Rückstandes in verdampfbaren Wertstoffen und/oder Lösungsmitteln unter Zusatz von bis zu 20 Gew.-% hochsiedenden Kohlenwasserstoffen, die unter Verdampfungsbedingungen der Wertstoffe inert sind, Erhitzen der Mischung auf Verdampfungstemperatur unter Vakuum, wobei die Wertstoffe verdampfen, abgezogen und kondensiert werden und der Rückstand als rieselfähiger Feststoff anfällt, wobei die Rückstandslösung auf ein bei Verdampfungstemperatur gehaltenes gerührtes Bett aus körnigem, festem Gut aufgebracht wird. Nachteilig hierbei ist der zusätzliche Einsatz von hoch siedenden Lösungsmitteln, die in einem weiteren Prozess aufgearbeitet werden müssen.

Die Hydrolyse von Isocyanat-Destillationsrückständen mit Wasser zwecks Rückgewinnung des Ausgangsamins, insbesondere bei der Herstellung von TDI, ist ein bereits seit längerer Zeit bearbeitetes Gebiet und beispielsweise in US 3,128,310, US 3,331,876, GB 795,639, DE 27 03 313 A1 und EP 1 935 877 A1 beschrieben. In den zitierten Verfahren wird Isocyanat-Destillationsrückstand bei erhöhtem Druck und erhöhter Temperatur mit Wasser hydrolysiert. Dabei wird ein Teil des Rückstandes in das Ursprungsamin umgewandelt, das nach entsprechender Aufarbeitung wieder in den Phosgenierprozess zurückgeführt werden kann und damit zu einer Rückstandsminimierung führt. Unbefriedigend bei diesen Verfahren ist, dass ein Teil des Wertproduktes Isocyanat wieder zum Ausgangsstoff hydrolisiert und erneut phosgeniert werden muss. Dadurch wird das im Rückstand enthaltene Isocyanat zwar einer sinnvollen stofflichen Verwertung zugeführt, jedoch wäre es wünschenswert, das Isocyanat als solches aus dem Rückstand zurückgewinnen zu können.

WO 2007/007887 von Mitsui Chemicals Polyurethanes, Inc. offenbart eine Isocyanat Rohproduktaufarbeitung, die neben der destillativen Isocyanatreinigung eine zweistufige Rückstandsaufkonzentrierung beschreibt. Gegebenenfalls kann das rückstandshaltige Gemisch anschließend einer Hydrolysereaktion unterworfen werden, die es ermöglicht, Einsatzamin zurückzugewinnen. Dazu wird das von Lösungsmittel befreite Isocyanat Rohprodukt einer Destillationskolonne zugeführt, wobei Isocyanat unter reduziertem Druck und erhöhter Temperatur abdestilliert wird und rückstandshaltiges Sumpfprodukt ausgetragen wird. Dieses Sumpfprodukt weist einen bevorzugten Rückstandsgehalt von 10-40 Gew.% bezogen auf das Isocyanat-/Rückstandsgemisch auf und wird mittels einer Pumpe in die zweite Stufe der Rückstandsaufkonzentrierung gefördert. Diese zweite Stufe besteht zum Beispiel aus einem unter erniedrigtem Druck betriebenen Dünnschichtverdampfer mit innenliegendem Kondensator. In diesem Verdampfer wird Isocyanat abgetrennt, kondensiert und ausgetragen, eine isocyanathaltige Rückstandsfraktion über eine Pumpe in eine Weiterbehandlung, wie zum Beispiel eine Rückstandshydrolyse, überführt. Die zweite Aufkonzentrierstufe reichert den Rückstandsgehalt bevorzugt auf 45-80 Gew.% an, wobei der Chlorgehalt dieser Fraktion bevorzugt nicht mehr als 1,5 Gew.% entspricht 15.000 ppm beträgt. Als Vorteile des beschriebenen Verfahrens werden die Abtrennung flüchtiger Chlorverbindungen bereits in der ersten Konzentrierstufe und die kurze Verweilzeit in der zweiten Stufe mit dem Ziel, die fortschreitende Viskositätszunahme durch thermisch induzierte Polymerisation zu unterdrücken, genannt. Als nachteilig einer solchen Verfahrensweise gilt, dass die angegebenen Chlorgehalte mit > 1Gew.% durchaus noch die thermisch induzierte Polymerisation in der zweiten Stufe (welche nicht Druck und Temperatur optimiert betrieben wird) protegieren und somit es zu einer nicht unerheblichen thermischen Rückstandbildung kommt, welche mit dem Verlust an Wertstoff (TDI) einhergeht. Die der Rückstandsaufkonzentrierung folgende weitere Aufarbeitung mittels einer Rückstandhydrolyse erscheint unter den angegebenen Konzentrationen unwirtschaftlich und aufwendig, da nur der noch vorhandenen Restgehalt an Wertstoff (TDI) mittels Hydrolyse in Einsatzamin zurück zu überführen ist, jedoch der gesamte erhaltende Rückstand der Hydrolyse unterworfen werden muß, was wiederum eine entsprechende Aufarbeitung erfordert.

Die DE 102 60 092 betrifft ein Verfahren zur Reinigung von Rohisocyanatströmen in dem an zwei verschieden Schritten rückstandshaltige Ströme abgetrennt werden. Hierzu wird der Rohisocyanatstrom zunächst in einer Verdampfung in einen rückstandshaltigen und einen gasförmigen Strom aufgetrennt. Während der rückstandshaltige Strom zum Beispiel in einem Knetertrockner oder Schaufeltrockner weiter von Isocyanatprodukt befreit wird, erfolgt eine destillative Trennung des gasförmigen Stromes in drei Teilströme, die im Wesentlichen aus Leichtsiederkomponenten, Isocyanatprodukt und einem weiteren rückstandshaltigen Strom bestehen. Der weitgehend aus Isocyanatprodukt bestehende Brüdenstrom aus der Trocknerstufe wird zusammen mit dem gasförmigen Strom aus der ersten Verdampfung, gegebenenfalls nach Kondensation, der genannten destillativen Trennung zugeführt. Ein Nachteil dieses Verfahrens ist, dass an zwei verschiedenen Stellen der Aufarbeitung Rückstandsströme entnommen und einer Verwertung zugeführt werden müssen.

In DE 102 60 093 wird ein Verfahren zur Abtrennung von Isocyanaten aus einem Reaktionsgemisch beschrieben, wobei das vom Lösungsmittel befreite Reaktionsgemisch in einer einzigen Trennstufe in drei Fraktionen getrennt wird. Dabei wird ein Kopfprodukt erhalten welches vorwiegend aus Chlorwasserstoff und Phosgen besteht und einer Vernichtung zugeführt wird. Am Seitenabzug der Kolonne wird TDI welches noch chlorierte Nebenprodukte enthält entnommen. Am Sumpf der Kolonne fallen TDI und TDI-haltiger Schwersieder an. Aus dieser Schwersieder-Fraktion wird in einer nachgelagerten Verdampfung nochmals rohes TDI gewonnen, welches dem aus dem Seitenabzug erhaltenden TDI zugeführt wird. Der im Sumpf erhaltene, teerartige Rückstand, wird ebenfalls einer Verbrennung zugeführt. Es wird jedoch nicht beschrieben, welche Rückstandkonzentrationen in der Eindampfung erreicht wird noch wird eine Konzentration an nachweisbaren NCO-Gehalt des teerartigen Rückstand erwähnt. Ferner wird nicht erwähnt, daß das erhaltene rohe TDI (aus Sumpf und Seitenabzug) noch weiteren Reinigungsschritten unterworfen wird, was angesichts des sehr hohen Gehaltes an chlorierten Nebenprodukten zwingend erforderlich ist und die Ausbeutebilanz nochmals negative beeinflußt wird. Somit bleibt offen, ob es sich bei dem beschriebenen Verfahren um ein wirtschaftlich bzw. großtechnisch praktikables Verfahren handelt.

EP 1 413 571 A1 und EP 1 371 633 A1 befassen sich mit der Optimierung der Aufarbeitung von TDI durch Einsatz einer Trennwandkolonne in der Destillation, was unter anderem eine Reduzierung des Gehalts an TDI im Sumpfprodukt zur Folge hat. Aber auch hier kann nicht verhindert werden, dass ein Isocyanat enthaltender Destillationsrückstand anfällt.

Die Aufgabe der vorliegenden Erfindung besteht darin, ein verbessertes Verfahren zur Aufbereitung von Phosgenierungsprodukten zur Verfügung zu stellen, insbesondere zur Gewinnung eines organischen Isocyanats aus einem das Isocyanat enthaltenden Phosgenierungsprodukt. Bei diesem Verfahren soll ein möglichst hoher Anteil der bei der Phosgenierung gebildeten organischen Isocyanate abgetrennt werden. Dabei soll der Verlust an durch unerwünschte chemische Reaktionen abgebauten organischen Isocyanaten und die Rückstandsmenge insgesamt möglichst gering gehalten werden und der verbleibende Rückstand möglichst leicht nach der Aufbereitung entfernbar sein.

Die Aufgabe konnte überraschenderweise gelöst werden, indem das Gemisch enthaltend TDI und höher siedender Komponenten mittels einer schonenden, zügigen, zweistufigen, kontinuierlichen Destillation zugeführt wird. Gegenstand der vorliegenden Erfindung ist somit ein Verfahren zur Gewinnung eines organischen Isocyanats aus einem das Isocyanat enthaltenden Phosgenierungsprodukt, umfassend die folgenden Schritte:

a) Aufarbeitung des Phosgenierungsprodukts, wobei die Aufarbeitung mindestens einen Destillationsschritt umfasst, bei dem eine erste Teilmenge des organischen Isocyanats als Destillat abgetrennt und ein Destillationsrückstand umfassend eine zweite Teilmenge des organischen Isocyanats erhalten wird,

b) Aufarbeitung des unter a) erhaltenen Destillationsrückstandes, wobei die Aufarbeitung mindestens einen Destillationsschritt umfasst, der bei einer Temperatur von bis zu 110 °C bei einem Druck von höchstens 1 mbar durchgeführt wird, wobei zumindest 50 Gew.-% der zweiten Teilmenge des organischen Isocyanats aus dem Destillationsrückstand abgetrennt werden.

Nach einer vorteilhaften Ausgestaltung des erfindungsgemäßen Verfahrens wird der Destillationsschritt der Aufarbeitung b) bei einer Temperatur von bis zu 90 °C und/ oder bei einem Druck von höchstens 1 mbar durchgeführt, insbesondere bei höchstens 0,8 mbar, vorzugsweise bei höchstens 0,5 mbar, besonders bevorzugt bei höchstens 0,3 mbar, ganz besonders bevorzugt bei höchstens 0,1 mbar. Auf diese Weise können noch schonendere Bedingungen für die Aufbereitung der organischen Isocyanate eingestellt werden, wodurch die Gefahr unerwünschter Reaktionen der Isocyanate weiter gemindert wird. Alle Druckangaben im Rahmen der vorliegenden Erfindung bezeichnen absolute Drücke.

Im Rahmen der vorliegenden Erfindung ist es weiter bevorzugt, dass die Verweilzeit des Destillationsrückstandes im Destillationsschritt der Aufarbeitung b) nicht länger als 30 Minuten beträgt, bevorzugt nicht länger als 15 Minuten. Durch die kurze Verweildauer kann die Menge an durch unerwünschte Reaktionen abgebauten organischen Isocyanaten gering gehalten werden.

Weiterhin können im Destillationsschritt der Aufarbeitung b) zumindest 60 Gew.-% der zweiten Teilmenge des organischen Isocyanats aus dem Destillationsrückstand abgetrennt werden, insbesondere wenigstens 70 Gew.-%.

Für Polymere werden üblicherweise Molmassenmittelwerte Mn und Mw und die Breite der Verteilung, D, angegeben. Als Maßzahl für die Breite einer Verteilung hat sich die Polydispersität D durchgesetzt. Die Größe Polydispersität ist insofern von Bedeutung, da beide Verteilungen die gleiche gewichtsmittlere Molmasse Mw aufweisen können und trotzdem eine unterschiedliche Verteilung (und damit unterschiedliche makroskopische Eigenschaften) haben können. Die Polydispersität ist definiert als Quotient aus Mw und Mn (D = Mw/Mn) und stellt eine unabhängige Berechnungsgrundlage zur Beurteilung der Molgewichtverteilung einer polymeren Verbindung dar. Als Kenngröße für die während der Aufkonzentrierung eintretende thermisch induzierte Polymerisation kann die Änderung des höhermolekularen Anteils vor und nach TDI-Abtrennung durch das ΔD beschrieben werden. Vorzugsweise wird während der Aufarbeitung b) eine thermisch induzierte Polymerisation des organischen Isocyanats weitestgehend unterdrückt, so dass für die Änderung der Polydispersität ΔD des höhermolekularen Anteils mit einer gewichtsmittleren Molmasse von mehr als 500 g/mol vor und nach der Aufarbeitung b) ΔD < 3,0 gilt, insbesondere ΔD < 2,0. Dies kann beispielsweise durch die vorgenannten kurzen Verweilzeiten, die genannten Temperaturen und/ oder Drücke erreicht werden.

Die im Rahmen des erfindungsgemäßen Verfahrens eingesetzten Phosgenierungsprodukte können auf jede dem Fachmann bekannte Weise erzeugt worden sein. Das Phosgenierungsprodukt wird beispielsweise durch Phosgenierung eines primären organischen Amins erhalten, wobei das Phosgenierungsprodukt das korrespondierende organische Isocyanat enthält. Dabei wird die Phosgenierung insbesondere in der Gasphase durchgeführt. Hierzu wird das organische Amin, insbesondere ein aromatisches Amin, mit Phosgen in der Gasphase bevorzugt nach der Lehre der EP 1 935 876 (adiabatische Reaktionsführung), besonders bevorzugt gemäß EP 2 196 455 oberhalb der Siedetemperatur der Amine in einem Reaktor enthaltend einen zur Strömungsrichtung im Wesentlichen rotationssymmetrischen Reaktionsraum zu rohem Isocyanat, dem Phosgenierungsprodukt, umgesetzt.

Als primäres organisches Amin kann ein aromatisches Di-, Tri- oder Polyamin verwendet werden, insbesondere Toluendiamin. Als organisches Isocyanat kommen insbesondere aromatische Di-, Tri oder Polyisocyanate in Betracht, insbesondere Toluylendiisocyanat. Jedoch können ebenso aliphatische und cyclische Amine beziehungsweise Isocyanate bei dem erfindungsgemäßen Verfahren eingesetzt werden. Vorzugsweise besteht das Toluylendiisocyanat zu wenigstens 80 Gew.-% aus dem Isomeren 2,4- Toluylendiisocyanat, insbesondere zu wenigstens 90 Gew.-%.

Der Gehalt an hydrolisierbarem Chlor (HC) gibt im Wesentlichen an, wieviel Chlorwasserstoff noch am Isocyanat in Form von Carbaminsäurechlorid gebunden ist. Ferner werden mit dem HC Gehalt auch Verbindungen erfasst, welche während der Phosgenierung als Nebenprodukte aus der Reaktion von Phosgen und dem bereits gebildetem Isocyanaten unter Abspaltung von $CO_2$ entstandenen Carbodiimide gebildet wurden, wie auch von H.J. Twichett in Chem. Soc. Rev., 1974,3, 209-230, postuliert. Es ist bekannt, dass Verbindungen, welche hydrolisierbares Chlor enthalten sich unter Einwirkung von Hitze in Chlorwasserstoff und Isocyanat zersetzten. Es ist weiterhin bekannt, dass Verbindungen, welche hydrolisierbares Chlor enthalten die thermisch induzierte Polymerisation protegieren, womit ein wesentlicher Nachteil in der Anwesenheit von Verbindungen, welche hydrolisierbares Chlor enthalten, besteht. Übliche Destillationssümpfe vergleichbarer Konzentrationen weisen einen Gehalt an hydrolisierbarem Chlor im Bereich von 10.000 ppm. Eine zuverlässige Methode zur Bestimmung des Gehaltes an hydrolisierbarem Chlor bietet die potentiometrische Titration, bei welcher die Probe zur Bestimmung des HC-Gehaltes mittels Methanol urethanisiert und mittels Wasser anschließend hydrolisiert wird. Das dabei gebildete ionogene Chlor wird nach Ansäuern mit Salpetersäure argentometrisch gegen eine Silbernitratmaßlösung titriert. Nach einer weiteren Ausführungsform des erfindungsgemäßen Verfahrens weist der nach der Aufarbeitung a) erhaltene Destillationsrückstand ohne weitere Aufarbeitung einen Gehalt an hydrolsierbaren Chlorverbindungen von weniger als 2000 ppm auf, insbesondere weniger als 1500 ppm.

Vorzugsweise weist bei dem erfindungsgemäßen Verfahren der nach Schritt b) erhaltene Destillationsrückstand einen NCO-Gehalt von wenigstens 20 Gew.-% auf, insbesondere wenigstens 25 Gew.-%. Die Ermittlung des NCO-Gehaltes erfolgt durch Titration gemäß DIN EN ISO 11 909. Die Rückstandskonzentration wird in mittels einer prozentualen Massenbilanz ermittelt, dazu wird die zu bestimmende Menge an Rückstandkonzentrat 30 min. bei 220°C und einem Druck von < 1 mbar von verdampfbaren Anteilen befreit, die Berechnung erfolgt nach:

$$\% \text{ Rückstandskonzentration} = \frac{(\text{g Rückstand (Auswaage)})}{(\text{g Einwaage zur Rückstandsbestimmung}*100)}$$

Der Verlust an nachweisbaren NCO-Gruppen gilt als Maß für die während der Aufkonzentrierung stattfindende thermisch induzierte Polymerisation, welche ggf. durch die Anwesenheit von chlorhaltigen Nebenkomponenten beschleunigt wird. Übersteigt die Abnahme des NCO-Gehalts die der Zunahme an Rückstand (Steigerung Rückstandskonzentration) kommt es zu der beschriebenen unerwünschten Polymerisation. Der Restgehalt an organischem Isocyanat des nach Schritt b) erhalten Destillationsrückstandes kann beispielsweise bis 30 Gew.-% betragen, insbesondere bis zu 25 Gew.-%, vorzugsweise bis zu 20 Gew.-%, zum Beispiel von 20 bis 30 Gew.-%.

Bei dem erfindungsgemäßen Verfahren kann der nach Schritt b) erhaltene Destillationsrückstand in aprotisch-polaren Lösungsmitteln einer Elutionskraft größer 0,55 e° mit $Al_2O_3$ als Adsorbens löslich sein, beispielsweise mit bis zu 90 Gew.-% bei Raumtemperatur. Unter der Elutionskraft e° ist die relative Adsorptionsenergie für jedes Lösungsmittel an Aluminiumoxid zu verstehen.

Als aprotisch-polare Lösungsmittel kommen insbesondere Ethlyacetat oder Aceton in Frage. Dies ist deshalb besonders vorteilhaft, weil auf hierdurch eine leichtere Entfernung der Destillationsrückstände als bei den bislang bekannten Verfahren möglich ist. Dies wird insbesondere durch die dem erfindungsgemäßen Verfahren zugrundeliegenden schonenden Aufbereitungsbedingungen bei verminderter Temperatur beziehungsweise Druck ermöglicht. So ist eine bevorzugte Ausgestaltung des erfindungsgemäßen Verfahrens dadurch gekennzeichnet, dass der nach Schritt b) erhaltene Destillationsrückstand in einem aprotisch-polaren Lösungsmittel einer Elutionskraft größer 0,55 e° mit $Al_2O_3$ als Adsorbens wenigstens teilweise gelöst und aus der Destillationsanlage entfernt wird, insbesondere in Ethlyacetat oder Aceton. Wie vorstehend ausgeführt wurde, können beispielsweise bis zu 90 Gew.-% des Destillationsrückstandes bei Raumtemperatur gelöst werden. Es werden dabei verfahrenstechnisch einfach handhabbare niedrigviskose Lösungen erhalten, die abgepumpt und beispielsweise bei Raumtemperatur gelagert werden können.

Die im Rahmen des erfindungsgemäßen Verfahrens durchgeführten Destillationsschritte können prinzipiell auf jede dem Fachmann bekannte Weise erfolgen. Bevorzugt wird der Destillationsschritt der Aufarbeitung a) und/ oder der Aufarbeitung b) mit Hilfe eines Verdampfers mit zwangsgefördertem Austrag durchgeführt, insbesondere mit Hilfe eines Durchlaufverdampfers, Fallfilmverdampfers, Langrohrverdampfers, Fallrohrverdampfers, Dünnschichtverdampfers oder Kurzwegverdampfers, besonders bevorzugt mit Hilfe eines Dünnschichtverdampfers oder Kurzwegverdampfers.

Beispiele:

Die vorliegende Erfindung wird im Folgenden anhand von Beispielen näher erörtert. In den Beispielen beziehen sich alle Prozentangaben auf das Gewicht. Die Ermittlung des NCO-Gehaltes der in den Beispielen und Vergleichsbeispielen beschriebenen Konzentrate erfolgte durch Titration gemäß DIN EN ISO 11 909. Die Reinheit und das Isomerenverhältnis des TDIs wurden gaschromatographisch bestimmt. Die Messungen erfolgten auf einem HP 5890 von Hewlett Packard mit FID-Detektor und der HP-Chemstation Software unter Verwendung einer HP35 Säule, als Referenz diente 2,4-TDI von Merk Artikel-No. 808264.

Der flüssige Produktstrom, das rohe Isocyanat, wird anschließend einer destillativen, im Allgemeinen mehrstufigen Aufarbeitung zugeführt, wobei gelöstes Phosgen sowie das Lösungsmittel abgetrennt werden. Diese destillative Aufarbeitung des rohen Isocyanats kann nach allgemein bekannten Methoden erfolgen. Beispiele sind in EP- 371 635 B1 und EP 1 413 571 B1 ausführlich beschrieben.

Bei EP 1 371 635 B1 Kapitel [0053] handelt es sich um eine zweistufige Aufarbeitung, bei der zunächst in einer ersten Destillationskolonne ein wesentlicher Teil des Lösungsmittels und die gesamten leichtsiedenden Komponenten, wie z. B. gelöstes Phosgen, aus dem rohen Isocyanat abgetrennt werden. Das am Kopf der Kolonne abgetrennte Lösungsmittel wird, gegebenenfalls durch weitere Reinigungsstufen von den leichtsiedenden Komponenten befreit, wieder dem Prozess der Phosgenierung zugeführt.

Im Sumpf der Kolonne wird ein Gemisch, bestehend aus dem restlichen Lösungsmittel, dem Produkt TDI und den höher siedenden Komponenten erhalten. In einer zweiten Destillationskolonne wird nun das restliche Lösungsmittel am Kopf der Kolonne abgetrennt. Im Seitenstrom der als Trennwandkolonne ausgeführten Destillation kann das reine TDI als Destillat entnommen werden. Im Sumpf der Trennwandkolonne wird ein Gemisch aus höhersiedenden Komponenten und TDI erhalten. Gemäß dem Beispiel EP 1 371 635 B1 liegt die Konzentration an höhersiedenden Komponenten bei 0,5 bis 15 Gew-%

Die destillative Aufarbeitung des nach dem erfindungsgemäßen Verfahren hergestellten Gemisches, bestehend aus TDI und höher siedenden Komponenten, erfolgt bevorzugt nach einer der drei nachfolgend dargestellten Beispielen:

**Beispiel 1**

Ein Gemisch enthaltend 90% TDI und 10% höher siedende Komponenten wird mit 840 ml/h Dosierrate einer Vakuumdestillation in einem Dünnschichtverdampfer (V2), mit vorgeschaltetem Vorverdampfer (V1), zugeführt. Dabei wird das Gemisch in der ersten Verdampferstufe (V1), bei 85°C (T(V1)) und 0,5 mbar (p(V1)), in 30% Sumpfablauf (S1) und 70% Destillat (D1) aufgetrennt. Das so erhaltene Destillat (D1) besteht zu 99,7% aus TDI w(TDI; D1) mit 88,6% Anteil an 2,4-Isomer. Der Sumpfablauf (S1) wird unmittelbar der zweiten Verdampferstufe (V2) zugeführt. In V2 wird bei 140°C (T(V2)) und 0,5 mbar (p(V2)), wiederum in einen Sumpfablauf S2 und Destillat D2 getrennt. Das erhaltene Destillat D2 besteht zu 99,3% aus TDI w(TDI; D2) mit 90,2% Anteil an 2,4-Isomer. Das erhaltene Konzentrat, Sumpfablauf S2, weist eine Rückstandskonzentration w(S2) = 69,9% und einen NCO Gehalt w(NCO; S2) = 29,4% auf.

**Beispiel 2**

Man verfährt wie in Beispiel 1 und führt das Gemisch enthaltend 90% TDI und 10% höher siedende Komponenten wird mit 780 ml/h Dosierrate einer Vakuumdestillation in einem Dünnschichtverdampfer (V2), mit vorgeschaltetem Vorverdampfer (V1), zu. Dabei wird das Gemisch in der ersten Verdampferstufe (V1), bei 80°C (T(V1)) und 0,4 mbar (p(V1)), in 25% Sumpfablauf (S1) und 75% Destillat (D1) aufgetrennt. Das so erhaltene Destillat (D1) besteht zu 99,6% aus TDI w(TDI; D1) mit 88,3% Anteil an 2,4 Isomer. Der Sumpfablauf (S1) wird unmittelbar der zweiten Verdampferstufe (V2) zugeführt. In V2 wird bei 145°C (T(V2)) und 0,4 mbar (p(V2)), wiederum in einen Sumpfablauf S2 und Destillat D2 getrennt. Das erhaltene Destillat D2 besteht zu 99,4% aus TDI w(TDI; D2) mit 90,6% Anteil an 2,4 Isomer. Das erhaltene Konzentrat, Sumpfablauf S2, weist eine Rückstandskonzentration w(S2) = 78,7% und einen NCO Gehalt w(NCO; S2) = 27,5% auf.

**Beispiel 3**

Man verfährt wie in Beispiel 1 und führt das Gemisch enthaltend 90% TDI und 10% höher siedende Komponenten wird mit 780 ml/h Dosierrate einer Vakuumdestillation in einem Dünnschichtverdampfer (V2), mit vorgeschaltetem Vorverdampfer (V1), zu. Dabei wird das Gemisch in der ersten Verdampferstufe (V1), bei 80°C (T(V1)) und 0,3 mbar (p(V1)), in 20% Sumpfablauf (S1) und 80% Destillat (D1) aufgetrennt. Das so erhaltene Destillat (D1) besteht zu 99,7% aus TDI w(TDI; D1) mit 88,1% Anteil an 2,4 Isomer. Der Sumpfablauf (S1) wird unmittelbar der zweiten Verdampferstufe (V2) zugeführt. In V2 wird bei 130°C (T(V2)) und 0,3 mbar (p(V2)), wiederum in einen Sumpfablauf S2 und Destillat D2 getrennt. Das erhaltene Destillat D2 besteht zu 99,2% aus TDI w(TDI; D2) mit 91,0% Anteil an 2,4 Isomer. Das erhaltene Konzentrat, Sumpfablauf S2, weist eine Rückstandskonzentration w(S2) = 62,9% und einen NCO Gehalt w(NCO; S2) = 30,0% auf.

**Vergleichsbeispiel**

Ein Gemisch enthaltend 13% ODB, 67% TDI und 20% höher siedende Komponenten wird mit einer Dosierrate von ca. 2300 L/h einem gerührten Kessel zugeführt. Der Kessel wird unter Vakuum (ca. 20-30 mbar) betrieben, die Sumpftemperatur des Kessels beträgt ca. 140-150°C. Unter diesen Bedingungen wird das Gemisch in Destillat und Sumpfablauf aufgetrennt. Das erhaltene Konzentrat weist eine Rückstandskonzentration von 55% und einen NCO Gehalt w(NCO) = 18,5% auf.

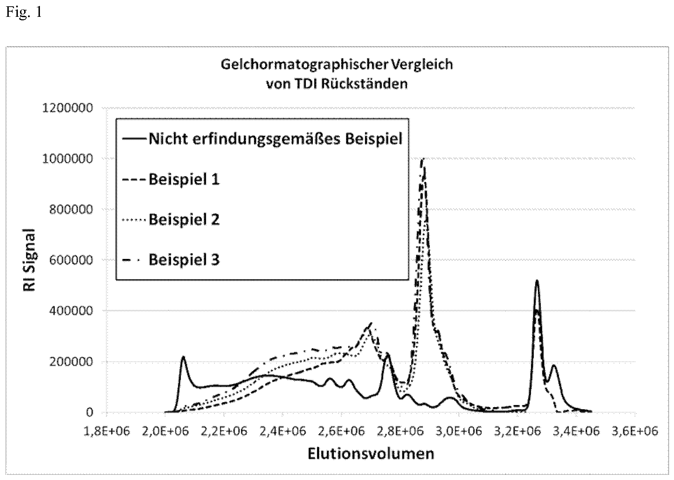

Die Konzentrate der Beispiele 1 bis 3 sowie des Vergleichsbeispiels wurden hinsichtlich ihrer Polydispersität mittels GPC untersucht. Der Bestimmungsbereich des verwendeten Säulensatzes lag im Bereich zwischen 100 und 20000 Dalton. Die Auswertung erfolgte mittels WIN GPC von Polymer Standard Services GmbH Mainz. Die rechnerische Auswertung der Ergebnisse ist in Tabelle 1 zusammengefasst und die grafische Auswertung in FIG. 1 dargestellt.

Wie einführend bereits beschrieben hat sich die Kennzahl D als unabhängige Größe zur Beschreibung der Polydispersität etabliert und gibt Auskunft über die Molgewichtsverteilung. Danach zeigen die erfindungsgemäß erzeugten Proben eine deutlich niedrigere Polydispersität als das Vergleichsbeispiel.

Tabelle 1, Ergebnisse der GPC - Auswertung

| TDI Rückstand | Mn | Mw | Mz | D |
|---|---|---|---|---|
| Vergleichsbeispiel (nicht erfindungsgemäß) (55 wt.-%) | 325 | 1480 | 4061 | 4,6 |
| Beispiel 1 (erfindungsgemäß) (71 wt.-%) | 344 | 619 | 1346 | 1,8 |
| Beispiel 2 (erfindungsgemäß) (81 wt.-%) | 488 | 810 | 1687 | 1,7 |
| Beispiel 3 (erfindungsgemäß) (62 wt.-%) | 499 | 736 | 1337 | 1,5 |

**Patentansprüche**

1. Verfahren zur Gewinnung eines organischen Isocyanats aus einem das Isocyanat enthaltenden Phosgenierungsprodukt, umfassend die folgenden Schritte:

a) Aufarbeitung des Phosgenierungsprodukts, wobei die Aufarbeitung mindestens einen Destillationsschritt umfasst, bei dem eine erste Teilmenge des organischen Isocyanats als Destillat abgetrennt und ein Destillationsrückstand umfassend eine zweite Teilmenge des organischen Isocyanats erhalten wird,
b) Aufarbeitung des unter a) erhaltenen Destillationsrückstandes, wobei die Aufarbeitung mindestens einen Destillationsschritt umfasst, der bei einer Temperatur von bis zu 110 °C bei einem Druck von höchstens 1 mbar durchgeführt wird, wobei zumindest 50 Gew.-% der zweiten Teilmenge des organischen Isocyanats aus dem Destillationsrückstand abgetrennt werden.

2. Verfahren nach Anspruch 1, **dadurch gekennzeichnet, dass** der Destillationsschritt der Aufarbeitung b) bei einer Temperatur von bis zu 90 °C und/ oder bei einem Druck von höchstens 1 mbar durchgeführt wird, insbesondere bei höchstens 0,8 mbar, vorzugsweise bei höchstens 0,5 mbar, besonders bevorzugt bei höchstens 0,3 mbar, ganz besonders bevorzugt bei höchstens 0,1 mbar.

3. Verfahren nach Anspruch 1 oder 2, **dadurch gekennzeichnet, dass** die Verweilzeit des Destillationsrückstandes im Destillationsschritt der Aufarbeitung b) nicht länger als 30 Minuten beträgt, bevorzugt nicht länger als 15 Minuten.

4. Verfahren nach einem der vorstehenden Ansprüche, **dadurch gekennzeichnet, dass** im Destillationsschritt der Aufarbeitung b) zumindest 60 Gew.-% der zweiten Teilmenge des organischen Isocyanats aus dem Destillationsrückstand abgetrennt werden, insbesondere wenigstens 70 Gew.-%.

5. Verfahren nach einem der vorstehenden Ansprüche, **dadurch gekennzeichnet, dass** während der Aufarbeitung b) eine thermisch induzierte Polymerisation des organischen Isocyanats weitestgehend unterdrückt wird, so dass für die Änderung der Polydispersität $\Delta D$ des höhermolekularen Anteils mit einer gewichtsmittleren Molmasse von mehr als 500 g/mol vor und nach der Aufarbeitung b) $\Delta D < 3,0$ gilt, insbesondere $\Delta D < 2,0$.

6. Verfahren nach einem der vorstehenden Ansprüche, **dadurch gekennzeichnet, dass** das Phosgenierungsprodukt durch Phosgenierung eines primären organischen Amins erhalten wird, wobei das Phosgenierungsprodukt das korrespondierende organische Isocyanat enthält, und wobei die Phosgenierung insbesondere in der Gasphase durchgeführt wird.

7. Verfahren nach Anspruch 6, **dadurch gekennzeichnet, dass** das primäre organische Amin ein aromatisches Di-, Tri- oder Polyamin, insbesondere Toluendiamin und das organische Isocyanat ein aromatisches Di-, Tri oder Polyisocyanat ist, insbesondere Toluylendiisocyanat.

8. Verfahren nach Anspruch 7, **dadurch gekennzeichnet, dass** das Toluylendiisocyanat zu wenigstens 80 Gew.-% aus dem Isomeren 2,4- Toluylendiisocyanat besteht, insbesondere zu wenigstens 90 Gew.-%.

9. Verfahren nach einem der vorstehenden Ansprüche, **dadurch gekennzeichnet, dass** der nach der Aufarbeitung a) erhaltene Destillationsrückstand ohne weitere Aufarbeitung einen Gehalt an hydrolsierbaren Chlorverbindungen von weniger als 2000 ppm aufweist, insbesondere von weniger als 1500 ppm.

10. Verfahren nach einem der vorstehenden Ansprüche, **dadurch gekennzeichnet, dass** der nach Schritt b) erhaltene Destillationsrückstand einen NCO-Gehalt von wenigstens 20 Gew.-% aufweist, vorzugsweise wenigstens 25 Gew.-%.

11. Verfahren nach einem der vorstehenden Ansprüche, **dadurch gekennzeichnet, dass** der Restgehalt an organischem Isocyanat des nach Schritt b) erhalten Destillationsrückstandes bis 30 Gew.-% beträgt, insbesondere bis zu 25 Gew.-%, vorzugsweise bis zu 20 Gew.-%.

12. Verfahren nach einem der vorstehenden Ansprüche, **dadurch gekennzeichnet, dass** der nach Schritt b) erhaltene Destillationsrückstand in einem aprotisch-polaren Lösungsmittel einer Elutionskraft größer 0,55 e° mit $Al_2O_3$ als Adsorbens wenigstens teilweise gelöst und aus der Destillationsanlage entfernt wird, insbesondere in Ethlyacetat oder Aceton.

13. Verfahren nach einem der vorstehenden Ansprüche, **dadurch gekennzeichnet, dass** der Destillationsschritt der Aufarbeitung a) und/ oder der Aufarbeitung b) mit Hilfe eines Verdampfers mit zwangsgefördertem Austrag, insbesondere mit Hilfe eines Durchlaufverdampfers, Fallfilmverdampfers, Langrohrverdampfers, Fallrohrverdampfers, Dünnschichtverdampfers oder Kurzwegverdampfers durchgeführt wird.

**Claims**

1. Process for obtaining an organic isocyanate from a phosgenation product comprising the isocyanate, comprising the steps of:

   a) working up the phosgenation product, wherein the workup comprises at least one distillation step where a first portion of the organic isocyanate is removed as distillate and a distillation residue comprising a second portion of the organic isocyanate is obtained,
   b) working up the distillation residue obtained in a), wherein the workup comprises at least one distillation step carried out at a temperature of up to 110°C at a pressure of not more than 1 mbar, wherein at least 50 wt% of the second portion of the organic isocyanate is removed from the distillation residue.

2. Process according to Claim 1, **characterized in that** the distillation step of the workup b) is carried out at a temperature of up to 90°C and/or at a pressure of not more than 1 mbar, in particular at not more than 0.8 mbar, preferably at not more than 0.5 mbar, particularly preferably at not more than 0.3 mbar, very particularly preferably at not more than 0.1 mbar.

3. Process according to either of Claims 1 and 2, **characterized in that** the residence time of the distillation residue in the distillation step of the workup b) is not longer than 30 minutes, preferably not longer than 15 minutes.

4. Process according to any of the preceding claims, **characterized in that** the distillation step of the workup b) removes at least 60 wt% of the second portion of the organic isocyanate from the distillation residue, in particular at least 70 wt%.

5. Process according to any of the preceding claims, **characterized in that** during the workup b) thermally induced polymerization of the organic isocyanate is very substantially prevented so that for the change in the polydispersity $\Delta D$ of the relatively high molecular weight fraction having a weight-average molar mass of more than 500 g/mol before and after the workup b) $\Delta D < 3.0$, in particular $\Delta D < 2.0$.

6. Process according to any of the preceding claims, **characterized in that** the phosgenation product is obtained by phosgenation of a primary organic amine, wherein the phosgenation product comprises the corresponding organic isocyanate and wherein the phosgenation is in particular carried out in the gas phase.

7. Process according to Claim 6, **characterized in that** the primary organic amine is an aromatic di-, tri- or polyamine, in particular toluenediamine, and the organic isocyanate is an aromatic di-, tri- or polyisocyanate, in particular tolylene diisocyanate.

8. Process according to Claim 7, **characterized in that** the tolylene diisocyanate comprises at least 80 wt%, in particular at least 90 wt%, of the 2,4-tolylene diisocyanate isomer.

9. Process according to any of the preceding claims, **characterized in that** the distillation residue obtained after the workup a) exhibits a content of hydrolyzable chlorine compounds of less than 2000 ppm, in particular less than 1500 ppm, without any further workup.

10. Process according to any of the preceding claims, **characterized in that** the distillation residue obtained after step b) has an NCO content of at least 20 wt%, preferably at least 25 wt%.

11. Process according to any of the preceding claims, **characterized in that** the residual content of organic isocyanate in the distillation residue obtained after step b) is up to 30 wt%, in particular up to 25 wt%, preferably up to 20 wt%.

12. Process according to any of the preceding claims, **characterized in that** the distillation residue obtained after step b) is at least partially dissolved and removed from the distillation plant in an aprotic polar solvent having an elution power greater than 0.55 e° with $Al_2O_3$ as the adsorbent, in particular in ethyl acetate or acetone.

13. Process according to any of the preceding claims, **characterized in that** the distillation step in the workup a) and/or the workup b) is carried out using a forced discharge evaporator, in particular using a single-pass evaporator, falling-film evaporator, long-tube evaporator, falling-tube evaporator, thin-film evaporator or short-path evaporator.


**Revendications**

1. Procédé d'obtention d'un isocyanate organique à partir d'un produit de phosgénation contenant l'isocyanate, comprenant les étapes suivantes :

   a) le traitement du produit de phosgénation, le traitement comprenant au moins une étape de distillation lors de laquelle une première partie de l'isocyanate organique est séparée en tant que distillat et un résidu de distillation comprenant une deuxième partie de l'isocyanate organique est obtenu,
   b) le traitement du résidu de distillation obtenu en a), le traitement comprenant au moins une étape de distillation qui est réalisée à une température de jusqu'à 110 °C à une pression d'au plus 1 mbar, au moins 50 % en poids de la deuxième partie de l'isocyanate organique étant séparé du résidu de distillation.

2. Procédé selon la revendication 1, **caractérisé en ce que** l'étape de distillation du traitement b) est réalisée à une température de jusqu'à 90 °C et/ou à une pression d'au plus 1 mbar, notamment d'au plus 0,8 mbar, de préférence d'au plus 0,5 mbar, de manière particulièrement préférée d'au plus 0,3 mbar, de manière tout particulièrement préférée d'au plus 0,1 mbar.

3. Procédé selon la revendication 1 ou 2, **caractérisé en ce que** le temps de séjour du résidu de distillation dans l'étape de distillation du traitement b) n'est pas supérieur à 30 minutes, de préférence pas supérieur à 15 minutes.

4. Procédé selon l'une quelconque des revendications précédentes, **caractérisé en ce que**, dans l'étape de distillation du traitement b), au moins 60 % en poids de la deuxième partie de l'isocyanate organique est séparé du résidu de distillation, notamment au moins 70 % en poids.

5. Procédé selon l'une quelconque des revendications précédentes, **caractérisé en ce qu'**une polymérisation induite thermiquement de l'isocyanate organique est essentiellement supprimée pendant le traitement b), de sorte que, pour la modification de la polydispersité ∆D de la fraction de masse moléculaire élevée ayant une masse molaire moyenne en poids de plus de 500 g/mol avant et après le traitement b), ∆D < 3,0, notamment ∆D < 2,0.

6. Procédé selon l'une quelconque des revendications précédentes, **caractérisé en ce que** le produit de phosgénation est obtenu par phosgénation d'une amine organique primaire, le produit de phosgénation contenant l'isocyanate organique correspondant, et la phosgénation étant notamment réalisée en phase gazeuse.

7. Procédé selon la revendication 6, **caractérisé en ce que** l'amine organique primaire est une di-, tri- ou polyamine aromatique, notamment la toluylène-diamine, et l'isocyanate organique est un di-, tri- ou polyisocyanate aromatique, notamment le diisocyanate de toluylène.

8. Procédé selon la revendication 7, **caractérisé en ce que** le diisocyanate de toluylène est constitué d'au moins 80 % en poids de l'isomère 2,4-diisocyanate de toluylène, notamment d'au moins 90 % en poids.

**9.** Procédé selon l'une quelconque des revendications précédentes, **caractérisé en ce que** le résidu de distillation obtenu après le traitement a) présente sans traitement supplémentaire une teneur en composés chlorés hydrolysables de moins de 2 000 ppm, notamment de moins de 1 500 ppm.

**10.** Procédé selon l'une quelconque des revendications précédentes, **caractérisé en ce que** le résidu de distillation obtenu après l'étape b) présente une teneur en NCO d'au moins 20 % en poids, de préférence d'au moins 25 % en poids.

**11.** Procédé selon l'une quelconque des revendications précédentes, **caractérisé en ce que** la teneur résiduelle en isocyanate organique du résidu de distillation obtenu après l'étape b) est de jusqu'à 30 % en poids, notamment de jusqu'à 25 % en poids, de préférence de jusqu'à 20 % en poids.

**12.** Procédé selon l'une quelconque des revendications précédentes, **caractérisé en ce que** le résidu de distillation obtenu après l'étape b) est au moins partiellement dissous dans un solvant aprotique polaire d'un pouvoir d'élution supérieur à 0,55 e° avec $Al_2O_3$ en tant qu'adsorbant et éliminé de l'unité de distillation, notamment dans de l'acétate d'éthyle ou de l'acétone.

**13.** Procédé selon l'une quelconque des revendications précédentes, **caractérisé en ce que** l'étape de distillation du traitement a) et/ou du traitement b) est réalisée à l'aide d'un évaporateur à déchargement forcé, notamment à l'aide d'un évaporateur à passage, d'un évaporateur à film tombant, d'un évaporateur à tube long, d'un évaporateur à tube de descente, d'un évaporateur à couche mince ou d'un évaporateur à court trajet.

Fig. 1

Gelchormatographischer Vergleich
von TDI Rückständen

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0548685 A2
- EP 0626368 A1
- EP 0699659 A2
- US 3128310 A
- US 3331876 A
- GB 795639 A
- DE 2703313 A1
- EP 1935877 A1
- WO 2007007887 A
- DE 10260092
- DE 10260093
- EP 1413571 A1
- EP 1371633 A1
- EP 1935876 A
- EP 2196455 A
- EP 371635 B1
- EP 1413571 B1
- EP 1371635 B1

**In der Beschreibung aufgeführte Nicht-Patentliteratur**

- **H.J. TWICHETT.** *Chem. Soc. Rev.,* 1974, vol. 3, 209-230